# EUROPEAN PATENT APPLICATION

(11) **EP 3 760 244 A1**
(43) Date of publication of application: **06.01.2021**
(21) Application number: 18907774.6
(22) Date of filing: 12.09.2018
(51) Int. Cl.: A61M 1/00

(54) **GAS COLLECTOR**

(30) Priority: 28.02.2018 RU 2018107424
(71) Applicant: Udartsev, Dobroslav Viktorovich, Novosibirsk, 630033 (RU)
(72) Inventor: Udartsev, Dobroslav Viktorovich, Novosibirsk, 630033 (RU)
(74) Representative: Jeck, Anton
(86) International application number: PCT/RU2018/050112
(87) International publication number: WO 2019/168433

(57) **Abstract**

Enhanced convenience of use is achieved due to the fact that the gas collector, which includes a device for the anus gas discharge, a tube connected to the said device with its first end, and a gas collection container connected to the second end of the tube, is characterized by the fact that the first end of the tube is connected to the gas relief device detachably to allow the first end of the tube and the anus gas relief device to be disconnected by applying force along the tube.

The force aimed at disconnecting the first end of the tube and the gas relief device can be to 5-7 kg. The gas collection container has a gas relief valve. This makes it possible for the user to release gases in a desired, ventilated location.

## Description

The invention relates to the area of satisfying human natural needs and can be used either at home or in hospitals.

The "*ANUS GAS OUTPUT DEVICE"* JPH0951942 *[2]* in the form of a gas relief tube which is inserted into the anus, **is well known.** The air relied opening is located in the external tail piece, which is outside the anus. The inlet gas opening is located at the outer periphery of the inserted internal cylindrical part, and the flatus input opening is connected with a degasing opening via the duct in the internal cylinder part.

The disadvantage of this device is inconvenience of use due to the connector location, which is placed outside and protrudes as refers to the anus, not allowing the patient to sit or lie on his or her back during the procedure.

**The closest** technical solution is "A three-chamber anal canal system" CN 205586346 [1] consisting of a gas collector, a tube and a gas collection container.

The user can sit or lie on his or her back with the device inserted into the anus.

The disadvantages of this system include the narrow range of application due to the impossibility of the tube disconnection and the fact that, in the emergency situations, the forces applied to the tube can lead to the fall of the gas collector out of the anus. The technical result of the proposed invention includes the extension of the application range and improved usability.

**The technical result** is achieved when the gas collector including the anus gas collector, the tube connected with its first end and the gas collection container connected with the second end of the tube, results in the fact that the first end of the tube is connected with the gas collector reversibly (with a possibility of disconnecting the first end of the tube and the anus gas collector by applying some force along the tube).

The separation force applied to the first end of the tube and the gas collector can be to 5-7 kg, which is sufficient to prevent emergency situations and is necessary to preserve the tube integrity.

At the first end of the tube (intended for the installation into the gas collector), an external retaining ring can be located allowing for the avoidance of any obstructions to the gas flow with sufficient strength and no additional fasteners.

The output opening of the gas collector can be provided with an internal groove, where the tube retaining ring is located, which simplifies the structure.

The gas collection container can be supplied with a flatus relief valve to prolong the running time and enhance user experience. The user can relief gases in a desired place with a ventilation system or outdoors.

**The gas collector** is shown in the picture (schematic view), where:
1 is the anus gas collector;
2 is the tube;
3 is the gas collection container;
4 is the retaining ring on the first end of the tube
5 is the gas collector internal groove;
6 is the second end of the tube;
7 is the gas relief valve.

**The device operates as follows** - tube (2) is connected with its first end to gas collector (1); at the second end (6) of the tube, there is gas collection container (3). There is retaining ring (4) at the first end of the tube, which is inserted into the groove (5) on the internal side of the gas collector output opening. The gas collection container can have some known attachments: flatus relief valve (7), container fasteners, voiding tools (not showed), etc. The first end of the tube with anus gas collector is connected by placing the retaining ring (4) at the first end of the tube into the groove (5).

**The technical result** - an expanded scope is achieved due to the fact that the gas collector can be used either as either an independent device or with a gas collection tube and a gas collection container. The technical result of enhancing the user experience is achieved due to the fact that when some force is applied to the tube, it is possible to disconnect the tube and the gas collector without any additional operations or without the gas collector falling out of the anus, which makes it possible to prevent emergency situations and ensure compliance with sanitary standards, including the situation when the user travels by transport or works.

**Industrial applicability.** The proposed engineering solution can be successfully used to produce convenient **gas collectors.**

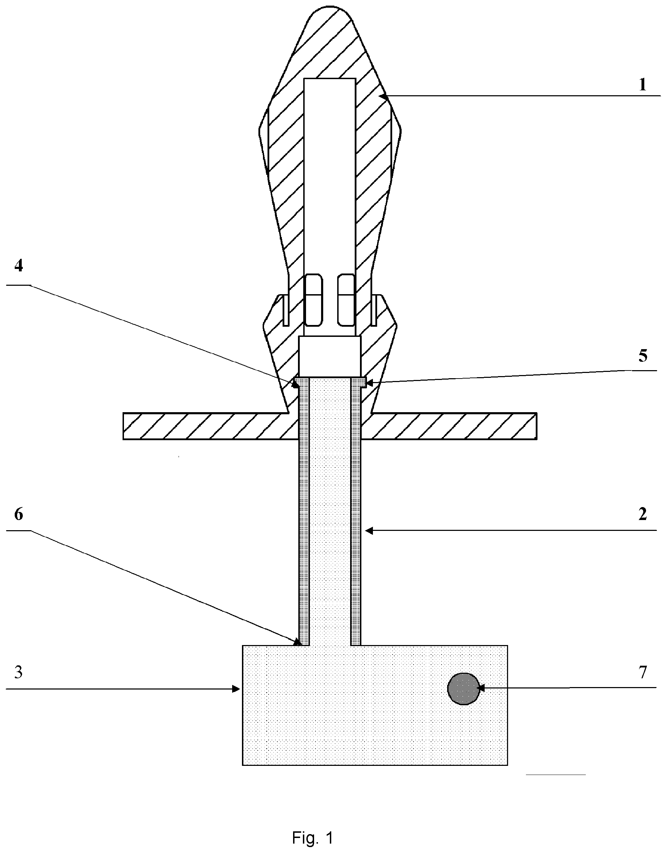

## Claims

1. The gas collector, including an anus gas collector, a tube connected with it with its first end and a gas container connected with second end, is **characterized by** the fact that the first end of the tube is connected with the gas collector reversibly, and the gas collecting container is provided with a gas relief valve.

2. The gas collector as of i.1 is **characterized by** the fact that the separation force for the first end of the tube and gas collector to 5-7 kg.

3. The gas collector as of i.1 is **characterized by** the fact that there is an external retaining ring on the first end of the tube.

4. The gas collector as of i.3 is **characterized by** the fact that the output opening of the gas collector is supplied with an internal groove, where the external retaining ring is installed.
